Europäisches Patentamt

European Patent Office

Office européen des brevets

(11) Veröffentlichungsnummer: **0 240 923**
**B1**

(12) **EUROPÄISCHE PATENTSCHRIFT**

(45) Veröffentlichungstag der Patentschrift:
04.07.90

(51) Int. Cl.⁵: **A61B 17/22, G10K 11/02**

(21) Anmeldenummer: **87104832.8**

(22) Anmeldetag: **01.04.87**

(54) **Stosswellenquelle mit piezokeramischer Druckquelle.**

(30) Priorität: **01.04.86 DE 3610818**

(43) Veröffentlichungstag der Anmeldung:
**14.10.87 Patentblatt 87/42**

(45) Bekanntmachung des Hinweises auf die Patenterteilung:
**04.07.90 Patentblatt 90/27**

(84) Benannte Vertragsstaaten:
**DE FR GB NL**

(56) Entgegenhaltungen:
**EP-A- 0 130 709**
**DE-A- 2 718 772**
**FR-A- 2 395 006**
**GB-A- 2 140 693**
**US-A- 4 281 550**

(73) Patentinhaber: **Siemens Aktiengesellschaft,
Wittelsbacherplatz 2, D-8000 München 2(DE)**

(72) Erfinder: **Naser, Georg, Dipl.-Ing. (FH), Karlstrasse 10,
D-8502 Zirndorf(DE)**
Erfinder: **Reichenberger, Helmut, Dr.,
Begonienstrasse 28, D-8501 Eckental(DE)**
Erfinder: **Schwark, Hubert, Heinrich-Hertz-Strasse 15,
D-8520 Erlangen(DE)**

# Beschreibung

Die Erfindung betrifft eine Stoßwellenquelle, insbesondere für einen Lithotripter, mit einer Ultraschall-Druckquelle für die Erzeugung eines Druckwellenimpulses und mit einer Vorlaufstrecke zur Weiterleitung des Druckwellenimpulses in Richtung auf einen Untersuchungskörper, wobei die Ultraschall-Druckquelle eine fokussierende piezoelektrische Ultraschallquelle ist.

Eine solche Stoßwellenquelle ist beispielsweise aus der deutschen Offenlegungsschrift 3 119 295 bekannt. Dort ist in einem Lithotripter eine Vielzahl von Ultraschall-Wandlerelementen eingesetzt, welche entweder auf elektronischem oder mechanischem Wege (dabei durch Formgebung) einen fokussierten Druckwellenimpuls abgeben. Zwischen den Wandlerelementen und dem Untersuchungskörper, in den der Druckwellenimpuls unter Bildung einer "Stoßwelle" zur Zerstörung eines Konkrements eingekoppelt wird, befindet sich die Vorlaufstrecke. Sie enthält Wasser.

Eine Stoßwellenquelle der eingangs genannten Art mit piezoelektrischen Wandlerelementen ist außerdem in der GB-A 2 140 693 beschrieben. Die Wandlerelemente sind in Form einer kugelkalottenförmigen Schale angeordnet, deren Höhe wenigstens 5 cm und deren Öffnungswinkel wenigstens 60° beträgt. Die Vorlaufstrecke ist mit einer Flüssigkeit oder einem weichen plastischen Material (z.B. Gießharz) gefüllt, wobei die akustische Impedanz des in der Vorlaufstrecke befindlichen Materials der des zu durchstrahlenden Gewebes möglichst exakt angepaßt ist.

In der EP-A 0 130 709 ist ein Ultraschallwandler für medizinisch/diagnostische Untersuchungen beschrieben. Dieser besitzt wenigstens ein Wandlerelement mit einer Abstrahlfläche für Ultraschallwellen, ein Teil zur Anpassung der akustischen Impedanz und ein Kontaktteil, das mit einem zu untersuchenden Objekt in Berührung gebracht wird und an der Abstrahlfläche des Ultraschallwandlers angebracht ist. Das Kontaktteil besteht aus einem plan-konkaven Verstärkungsteil und einer mit diesem verbundenen bikonvexen akustischen Linse. Das Kontaktteil besteht aus einem polymeren Werkstoff, dessen akustische Impedanz sehr dicht bei der von menschlichem Gewebe liegt.

Weiter ist in der FR-A 2 395 006 ein vor der Applikation eines Ultraschallwandlers im Applikationsbereich auf die Körperoberfläche aufzutragendes Ultraschallgel hoher Viskosität beschrieben, das als wesentliche Bestandteile Wasser und Glyzerin enthält.

Nachteilig beim Einsatz von piezokeramischen Wandlerelementen ist, daß bereits an der Druckquelle ein relativ steiler Druckwellenimpuls entsteht. Dadurch erfolgt der Übergang oder die Umwandlung des Druckwellenimpulses zur eigentlichen "Stoßwelle" bereits relativ rasch, d.h. in einem unerwünscht großen Abstand zum Fokus. Unerwünscht ist dieser große Abstand deswegen, weil die "Stoßwelle" gegenüber dem Druckwellenimpuls mit einer erheblichen zusätzlichen Dämpfung aufgrund nichtlinearer Effekte belastet ist. Diese nichtlineare Dämpfung ist im Stoff der Vorlaufstrecke, insbesondere auch in Wasser, sehr viel höher als die normale lineare Dämpfung. Die "Stoßwelle" verliert dadurch bis zum Erreichen des Fokus beträchtlich an Energie. Weiterhin hat die sehr kurze Pulsdauer der "Stoßwelle" eine sehr kleine Wellenlänge der Grundschwingung zur Folge. Damit ergibt sich eine sehr kleine Fokuszone mit einem entsprechend geringen Wirkungsvolumen für die Zerstörung des Konkrements.

Aufgabe der Erfindung ist es, eine Stoßwellenquelle der eingangs genannten Art anzugeben, bei der die Ausbildung der Stoßwelle erst später, d.h. an einem näher am Fokus liegenden Ort erfolgt.

Diese Aufgabe wird erfindungsgemäß dadurch gelöst, daß die Vorlaufstrecke einen Stoff mit einer akustischer Impedanz enthält, die wenigstens gleich der von Äthylenglykol ist, und daß die Ultraschall-Druckquelle einen Durchmesser von mindestens 10 cm und einen effektiven Krümmungsradius bis maximal etwa 20 cm besitzt und mit einer Ansteuerspannung mit einer Frequenz von weniger als 500 kHz betrieben ist.

Vorteil dieser Maßnahmen ist es, daß sich der Druckwellenimpuls erst in einem näher zum Fokus liegenden Abstand zur eigentlichen "Stoßwelle" ausbildet. Dadurch wird der Bereich, in dem die nichtlineare Dämpfung wirksam ist, reduziert. Der Abstand soll allerdings nicht so klein werden, daß er eine halbe Wellenlänge der Grundschwingung des Druckwellenimpulses unterschreitet. In einem solchen Fall wäre aufgrund von Beugungsbegrenzungen die Bildung einer "Stoßwelle" nicht mehr möglich.

Es läßt sich mit den beschriebenen Maßnahmen eine kurze, leicht handhabbare Stoßwellenquelle auf Piezokeramik-Basis mit erhöhter Energieabgabe aufbauen.

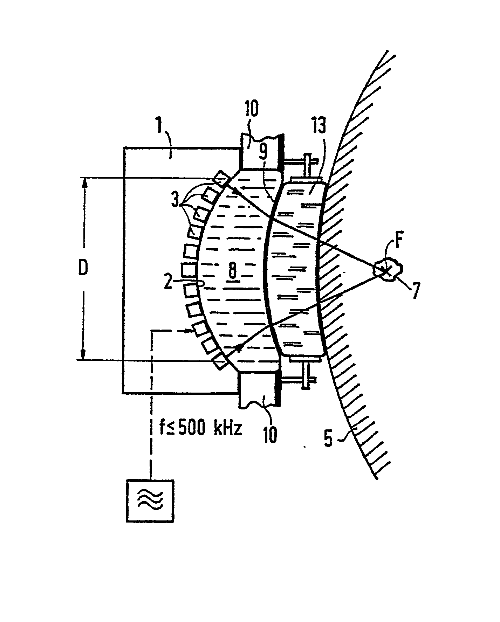

Weitere Ausgestaltungen und Vorteile der Erfindung ergeben sich aus der Beschreibung eines Ausführungsbeispiels anhand der Figur.

Die Figur zeigt eine piezoelektrische Druckquelle 1 mit konkav gekrümmter Abstrahlfläche 2, die mittels eines piezokeramischen Stoffes realisiert ist. Die Druckquelle 1 kann insbesondere eine Anzahl piezokeramischer Schwinger oder Ultraschall-Wandlerelemente 3 auf einer Halbkugel-Oberfläche 2 umfassen. Die piezokeramischen Schwinger 3 weisen (unter Berücksichtigung der Brechung) auf einen Fokus F, der innerhalb des Körpers (Untersuchungskörpers) eines Patienten 5 liegt. Die Druckquelle 1 ist so justiert, daß der Fokus F im Bereich eines zu zerstörenden Konkrements 7, z.B. eines Nierensteins, liegt.

Die piezokeramischen Schwinger 3 grenzen an eine konvex-konkave Vorlaufstrecke 8 an, welche frontseitig mit einer Abschlußmembran 9 und randseitig von einem Gehäuse 10 begrenzt ist. Die Abschlußmembran 9, das Gehäuse 10 und die piezokeramischen Schwinger 3 auf der Abstrahlfläche 2 begrenzen somit den Raum der Vorlaufstrecke 8. Diese ist mit einem Stoff mit relativ hoher Schallgeschwindigkeit v und relativ hoher Dichte φ gefüllt.

Diese Angaben bezüglich der Schallgeschwindigkeit v und der Dichte φ sind im Verhältnis zu den Werten von Wasser bewertet.

Somit liegt auch die Impedanz ($\varphi$ x v) höher als die von Wasser.

Als Stoff kommt insbesondere Glyzerin, aber auch ein anderer mehrwertiger Alkohol wie Äthylenglykol, in Frage.

An die konkave äußere Seite der Abschlußmembran 9 schließt sich ein Ankoppelkörper 13 an. Der Ankoppelkörper 13, z.B. aus einem Ankoppelgel, grenzt mit seiner freien Stirnseite an den Patienten 5. Für gewisse Anwendungen kann der Ankoppelkörper 13 auch durch eine Ankoppelpaste für Ultraschallwellen oder ein anderes Medium ersetzt werden.

Im folgenden wird eine Glyzerinfüllung mit einer Wasserfüllung in der Vorlaufstrecke 8 verglichen. Aufgrund der hier betrachteten Glyzerinfüllung in der Vorlaufstrecke 8 bildet sich der von den einzelnen Schwingern 3 erzeugte Druckwellenimpuls erst an einer Stelle zur "Stoßwelle" aus, die näher zum Fokus F gelegen ist als bei einer üblichen Wasserfüllung. Der Druckwellenimpuls bleibt dadurch länger im Bereich der linearen Dämpfung. Dadurch werden Verluste aufgrund der nichtlinearen Dämpfung der "Stoßwelle" reduziert. Diese Reduktion hat zur Folge, daß die piezokeramischen Schwinger 3 mit einer kleineren Ansteuerspannung als üblich betrieben werden können. Die Amplitude des erzeugten Druckwellenimpulses kann also zurückgenommen werden. Dieses wiederum bringt den Vorteil mit sich, daß die Lebensdauer der piezokeramischen Schwinger 3 erhöht und die Belastung des Patienten 5 durch akustische Energie reduziert ist.

Andererseits kann bei vorgegebener, z.B. durch die Spannungsfestigkeit der piezokeramischen Schwinger 3 begrenzter maximaler Ansteuerspannung durch die hier angegebene Füllung der Vorlaufstrecke 8 mit einem Stoff relativ hoher akustischer Impedanz ($\varphi$ x v) eine "Stoßwelle" erzeugt werden, die eine höhere Energie und damit eine höhere Wirksamkeit besitzt.

Bei der piezokeramischen Druckquelle 1 kann es sich um ein einziges großes Piezoelement oder aber – wie gezeigt – um eine Vielzahl von einzelnen Piezoelementen oder Piezoschwingern 3 handeln. Die Druckquelle 1 kann an ihrer Abstrahlfläche 2 konkav gekrümmt sein. Abweichend davon kann sie auch als ebener Schwinger (nicht gezeigt) ausgebildet sein, dem eine mechanische Fokussierungseinrichtung (nicht gezeigt), wie z.B. eine Linse oder ein Reflektor, zugeordnet ist. Alternativ kann die Fokussierungseinrichtung ausschließlich durch eine plan-konkave Formgebung (nicht gezeigt) der Vorlaufstrecke 8 gebildet werden. Die Druckquelle 1 kann aber auch aus einer Anzahl von Ultraschall-Wandlerelementen 3, insbesondere in (bevorzugt ebener) konzentrisch ringförmiger Anordnung, aufgebaut sein, wobei diese Wandlerelemente 3 zeitverzögert angesteuert werden (nicht gezeigt); dabei kommt eine elektronische Fokussierung zustande, die an sich aus der medizinischen Ultraschalltechnik bekannt ist.

Die dargestellte konkave piezokeramische Druckquelle 1 hat einen Durchmesser D größer etwa 10 cm und einen Krümmungsradius R bis maximal etwa 20 cm. Sie wird zur Erzeugung des Druckwellenimpulses mit einer Ansteuerspannung mit einer Frequenz von weniger als 500 kHz betrieben, was in der Figur durch einen Ansteuer-Block symbolisiert ist. Handelt es sich um eine Druckquelle 1 mit zugeordneter Fokussierungseinrichtung oder mit zeitverzögerter Elementarsteuerung, so soll der hierbei auftretende "effektive Krümmungsradius", der die Fokussierung bestimmt, unterhalb von etwa 20 cm liegen.

Die niedere Frequenz von kleiner oder gleich etwa 500 kHz hat zur Folge, daß einerseits in einem weiten Bereich des Ausbreitungsweges die Verluste durch lineare Dämpfung, die bekanntlich mit steigender Frequenz zunehmen, gering gehalten werden, und daß andererseits der Punkt, an dem sich die Stoßwelle ausbildet, in Richtung auf den Fokus F verlagert wird.

Eine andere Verwendung der beschriebenen Stoßwellenquelle als die Konkrementzerstörung ist die Behandlung von im Körper oder auf der Haut eines Patienten liegender Tumore mit Stoßwellen.

**Patentansprüche**

1. Stoßwellenquelle, insbesondere für einen Lithiotripter, mit einer Ultraschall-Druckquelle (1) für die Erzeugung eines Druckwellenimpulses und mit einer Vorlaufstrecke (8) zur Weiterleitung des Druckwellenimpulses in Richtung auf einen Untersuchungskörper (5), wobei die Ultraschall-Druckquelle (1) eine fokussierende piezoelektrische Ultraschallquelle ist, wobei die Vorlaufstrecke einen Stoff mit einer akustischen Impedanz enthält, die größer oder gleich der akustischen Impedanz von Äthylenglykol ist, und wobei die Ultraschall-Druckquelle (1) einen Durchmesser (D) von mindestens 10 cm und einen effektiven Krümmungsradius (R) bis maximal etwa 20 cm besitzt und mit einer Ansteuerspannung mit einer Frequenz von weniger als 500 kHz betrieben ist.

2. Stoßwellenquelle nach Anspruch 1, **dadurch gekennzeichnet,** daß der Stoff in der Vorlaufstrecke (8) Glyzerin oder ein anderer mehrwertiger Alkohol ist.

3. Stoßwellenquelle nach Anspruch 1 oder 2, **dadurch gekennzeichnet,** daß die Druckquelle (1) eine Anzahl von piezokeramischen Ultraschall-Wandlerelementen (3) umfaßt.

4. Stoßwellenquelle nach Anspruch 1 oder 2, **dadurch gekennzeichnet,** daß die Druckquelle (1) ein einziges piezokeramisches Ultraschall-Wandlerelement umfaßt.

5. Stoßwellenquelle nach einem der Ansprüche 1 bis 4, **dadurch gekennzeichnet,** daß die Vorlaufstrecke (8) eine konvex-konkave Gestalt hat.

6. Stoßwellenquelle nach einem der Ansprüche 1 bis 5, **dadurch gekennzeichnet,** daß zwischen der Vorlaufstrecke (8) und dem Untersuchungskörper (5) ein Ankoppelkörper (13) vorgesehen ist.

7. Stoßwellenquelle nach einem der Ansprüche 1 bis 6, **dadurch gekennzeichnet,** daß die Abstrahlfläche (2) der Druckquelle (1) konkav ist.

8. Stoßwellenquelle nach einem der Ansprüche 1 bis 7, **dadurch gekennzeichnet,** daß die Abstrahlfläche (2) der Druckquelle (1) eben ist, und daß der Druckquelle (1) eine mechanische oder elektrische Fokussiereinrichtung zugeordnet ist.

**Revendications**

1. Générateur d'ondes de choc notamment pour un lithotriteur, comportant une source de pression à ultrasons (1) pour la production d'une impulsion d'onde de pression et une section de parcours amont (8) servant à retransmettre l'impulsion d'onde de pression en direction d'un corps à examiner (5), la source de pression à ultrasons (1) étant une source ultrasonore piézoélectrique réalisant une focalisation, et dans lequel la section de parcours amont contient une substance possédant une impédance acoustique supérieure ou égale à l'impédance acoustique de l'éthylène-glycol et dans laquelle le générateur d'ondes à ultrasons (1) possède un diamètre (D) égal à au moins 10 cm et un rayon de courbure effectif (R) atteignant au maximum environ 20 cm et est alimenté par une tension de commande possédant une fréquence inférieure à 500 kHz.

2. Générateur d'ondes de choc suivant la revendication 1, caractérisé par le fait que la substance présente dans la section de parcours amont (8) est de la glycérine ou un autre alcool polyvalent.

3. Générateur d'ondes de choc suivant la revendication 1 ou 2, caractérisé par le fait que la source de pression (1) comporte un certain nombre d'éléments transducteurs piézocéramiques à ultrasons (3).

4. Générateur d'ondes de choc suivant la revendication 1 ou 2, caractérisé par le fait que la source de pression (1) comporte un seul élément transducteur piézocéramique à ultrasons.

5. Générateur d'ondes de choc suivant l'une des revendications 1 à 4, caractérisé par le fait que la section de parcours amont (8) possède une configuration convexe-concave.

6. Générateur d'ondes de choc suivant l'une des revendications 1 à 5, caractérisé par le fait qu'entre la section de parcours amont (8) et le corps à examiner (5), il est prévu un corps de couplage (13).

7. Générateur d'ondes de choc suivant l'une des revendications 1 à 6, caractérisé par le fait que la surface rayonnante (2) de la source de pression (1) est concave.

8. Générateur d'ondes de choc suivant l'une des revendications 1 à 7, caractérisé par le fait que la surface rayonnante (2) de la source de pression (1) est plane et qu'un dispositif de focalisation mécanique ou électrique est associé à la source de pression (1).

**Claims**

1. Shock wave source, in particular for a lithotripter, having an ultrasonic pressure source (1) for producing a pressure wave pulse and having a transmission path (8) for conveying the pressure wave pulse in the direction of a body being examined (5), wherein the ultrasonic pressure source (1) is a focussing piezo-electric ultrasonic source, the transmission path contains a substance with an acoustic impedance which is greater than or equal to the acoustic impedance of ethylene glycol, and the ultrasonic pressure source (1) has a diameter (D) of at least 10 cm and an effective radius of curvature (R) up to approximately 20 cm maximum, and is operated by a control voltage at a frequency of less than 500 kHz.

2. Shock wave source according to claim 1, characterised in that the substance in the transmission path (8) is glycerine or another multivalent alcohol.

3. Shock wave source according to claim 1 or 2, characterised in that the pressure source (1) includes a number of piezoceramic ultrasonic transducer elements (3).

4. Shock wave source according to claim 1 or 2, characterised in that the pressure source (1) includes a single piezoceramic ultrasonic transducer element.

5. Shock wave source according to one of claims 1 to 4, characterised in that the transmission path (8) has a convex-concave shape.

6. Shock wave source according to one of claims 1 to 5, characterised in that a coupling member (13) is provided between the transmission path (8) and the body being examined (5).

7. Shock wave source according to one of claims 1 to 6, characterised in that the emission surface (2) of the pressure source (1) is concave.

8. Shock wave source according to one of claims 1 to 7, characterised in that the emission surface (2) of the pressure source (1) is flat, and in that a mechanical or electrical focussing device is associated with the pressure source (1).